# EUROPEAN PATENT SPECIFICATION

(11) **EP 3 743 042 B1**
(45) Date of publication and mention of the grant of the patent: **03.06.2026**
(21) Application number: 19704867.1
(22) Date of filing: 07.01.2019
(51) Int. Cl.: A61K 9/00, A61K 9/16, A61K 9/48, A61K 9/50, A61K 31/045, A61K 31/167

(54) **PERFORATED CAPSULES**
PERFORIERTE KAPSELN
CAPSULES PERFORÉES

(30) Priority: 22.01.2018 US 201862620101 P
(43) Date of publication of application: 02.12.2020
(73) Proprietor: Kenvue Brands LLC, Summit, NJ 07901 (US)
(72) Inventor: BAGCHI, Saumitra, Fort Washington Pennsylvania 19034 (US); MCNALLY, Gerard P., Fort Washington Pennsylvania 19034 (US)
(74) Representative: Carpmaels & Ransford LLP
(86) International application number: PCT/IB2019/050105
(87) International publication number: WO 2019/142063

(56) References cited:
- CN-A- 105 168 000
- CN-U- 205 411 711
- JP-A- 2006 290 821
- US-A1- 2016 106 681
- CIPER M ET AL: "Modified conventional hard gelatin capsules as fast disintegrating dosage form in the oral cavity", EUROPEAN JOURNAL OF PHARMACEUTICS AND BIOPHARMACEUTICS, ELSEVIER SCIENCE PUBLISHERS B.V., AMSTERDAM, NL, vol. 62, no. 2, 1 February 2006 (2006-02-01), pages 178 - 184, XP027998057, ISSN: 0939-6411, [retrieved on 20060201], DOI: 10.1016/J.EJPB.2005.08.014
- K MALLESWARI: "MICROENCAPSULATION: A REVIEW A NOVEL APPROACH IN DRUG DELIVERY", EUROPEAN JOURNAL OF PHARMACEUTICAL AND MEDICAL RESEARCH, 1 June 2016 (2016-06-01), pages 186 - 194, XP055573793, ISSN: 2394-3211

## Description

### FIELD OF THE INVENTION

The present invention relates to hard capsules that are perforated and contain a pharmaceutically acceptable fill.

### BACKGROUND OF THE INVENTION

Hard gelatin capsules were traditionally a popular dosage form for prescription and over-the-counter (OTC) drugs, and many patients prefer capsules over tablets, perceiving them as being easier to swallow. Capsules are hard shell compartments made of two halves, including a body and a cap, wherein the cap partially and snugly overlaps with the body to enclose a dosable drug ingredient therein. The enclosed dosable ingredient is most often a powder, liquid, paste or similar nonsolid form. Capsules have long been recognized as a preferred dosage form for the oral delivery of active ingredients, which may be in the form of powder, liquid or granules of different compositions, for delivery to the gastro-intestinal tract of a human. Advantages of capsules as a dosage form include the variety of shapes and color combinations (including different colored caps and bodies), enhancing their unique identification, their glossy elegant appearance, and their easy swallowability. One type of commonly used capsule is a two-piece hard shell capsule, typically made from gelatin, starch, or cellulose derivatives. The hard-shell capsule typically comprises a longer body having an outside diameter, and a relatively shorter cap having an inside diameter that will just fit over the outside diameter of the body. The cap fits snugly over the body, creating an overlapping portion of the capsule.

Generally, empty hard-shell capsules are produced by a conventional dip-molding process such as that which is described on page 182 of "Pharmaceutical Dosage Forms and Drug Delivery Systems, 7th Ed.", (1999) by Howard C. Ansel, Loyd V. Allen Jr., and Nicholas G. Popovich, published by Lippincott Williams & Wilkins, Baltimore, Md. Consumers have found that such capsules are aesthetically pleasing, easy to swallow and mask the medicine taste of the drug contained therein. In addition, the bodies and caps of such capsules are often produced in different colors, resulting in a bi-colored capsule product having enhanced aesthetic appeal, as well as improved product identification and brand recognition by consumers. Many patients preferred capsules over coated or uncoated tablets, prompting pharmaceutical manufacturers to market certain products in capsule form even when they were also available in tablet form.

Several materials are known to be used to form the shell. Gelatin has been adopted as the main material of these capsules due to its excellent characteristic as a gelatinizer. The gelatin dissolves under high concentration into water of a high temperature and quickly gels at room temperature conditions of about 25°C. The thickness of the film made by the gelatin becomes uniform. However, gelatin is one of the proteins derived from animals; most commonly from the bones of bovine animals; and can be viewed as unstable from a chemical viewpoint due to its tendency to cross-link, which can slow the dissolution rate, can be microbally unstable, and has a risk of TSE. As a result, several materials have been examined as a substitute for the gelatin in two-piece hard capsules. Hydroxypropylmethyl cellulose (HPMC) or hypromellose is used as an alternative material for two-piece capsule. HPMC capsules have been developed for both pharmaceutical products and dietary supplements.

Ciper, et al., Modified conventional hard gelatin capsules as fast disintegrating dosage form in the oral cavity, European Journal of Pharmaceutics and Biopharmaceutics 62 (2006), 178-185, discloses perforated capsules with 2-20 holes made by a needle. The holes are 25-50 um. The disintegration time was more the half of the non-perforated capsule. The capsules are designed to dissolve in the mouth. US 2016/0106681 A1 relates to: *"A vegetarian pore-sealing capsule [...]. The pore-sealing capsule may release active substances in a gastrointestinal tract, including stomach, duodenum, jejunum, ileum and colon, under control. The pore-sealing capsule includes a capsule shell having one or more pores and a pore-sealing material for sealing at least one of the pores. When the pore-sealing capsule is located at a destination inside the gastrointestinal tract, the pore-sealing material will leave the pores."*

It is one object of the present invention to provide a swallowable capsule that remains intact in the mouth and having faster disintegration and/or dissolution times in the stomach relative to the commercially available capsules.

### SUMMARY OF THE INVENTION

The present invention is perforated hard capsule including at least one perforation having a first dimension and containing at least one particle with a dimension larger than the perforation in the capsule wherein the capsule and the at least one particle are not dissolvable in the mouth. The capsule can contain a plurality of perforations. The perforations can have a first dimension of about 0.1mm to about 5.0 mm or a first dimension from about 0.5 mm to about 3.00 mm.

### BRIEF DESCRIPTION OF THE DRAWINGS

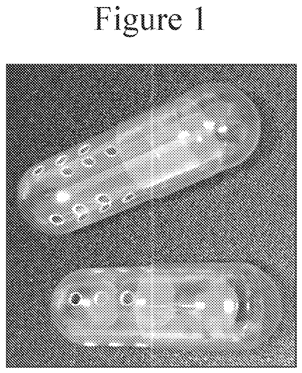

Figure 1 is a photograph of two capsules with perforations.

### DETAILED DESCRIPTION OF THE INVENTION

As used herein, the term "dosage form" applies to any solid object, semi-solid, or liquid composition designed to contain a specific pre-determined amount (dose) of a certain ingredient, for example an active ingredient as defined below. Suitable dosage forms may be pharmaceutical drug delivery systems, including those for oral administration, buccal administration, rectal administration, topical or mucosal delivery, or subcutaneous implants, or other implanted drug delivery systems; or compositions for delivering minerals, vitamins and other nutraceuticals, oral care agents, flavorants, and the like. Preferably the dosage forms of the present invention are considered to be solid, however they may contain liquid or semi-solid components. In a particularly preferred embodiment, the dosage form is an orally administered system for delivering a pharmaceutical active ingredient to the gastro-intestinal tract of a human. In another preferred embodiment, the dosage form is an orally administered "placebo" system containing pharmaceutically inactive ingredients, and the dosage form is designed to have the same appearance as a particular pharmaceutically active dosage form, such as may be used for control purposes in clinical studies to test, for example, the safety and efficacy of a particular pharmaceutically active ingredient

Examples of active ingredients useful in the present invention include propionic acid derivatives, which are a well known class of analgesic compounds. As used herein propionic acid derivatives are understood to include, but are not limited to, ibuprofen, naproxen, benoxaprofen, naproxen sodium, flurbiprofen, fenoprofen, fenbuprofen, ketoprofen, indoprofen, pirprofen, carpofen, oxaprofen, pranoprofen, microprofen, tioxaprofen, suproprofen, alminoprofen, tiaprofenic acid, fluprofen and bucloxic acid. The structural formula is set forth in U.S. Pat. No. 4,923,898. Propionic acid derivatives as defined herein are defined as pharmaceutically acceptable analgesics/non-steroidal anti-inflammatory drugs having a free --CH(CH3)COOH or --CH2 CH2 COOH or a pharmaceutically acceptable salt group, such as --CH(CH3)COO--Na+ or CH.sub.2 CH2 COO--Na+, which are typically attached directly or via a carbonyl functionality to an aromatic ring system.

Propionic acid derivatives are typically administered on a daily basis, with the daily dose ranging from about 25 to about 2000 milligrams, preferably from about 100 to about 1600 milligrams and most preferably from about 100 to about 1200 milligrams. Ibuprofen is typically administered on a daily basis, with a daily dose ranging from about 50 to about 2000 milligrams, preferably from about 100 to about 1600 milligrams and most preferably from about 200 to about 1200 milligrams. In one embodiment of this invention each individual hard shell liquid filled capsule contains about 50 to about 400 milligrams or about 100 milligrams to about 200 milligrams of ibuprofen.

Ibuprofen is a widely used, well known non-steroidal anti-inflammatory propionic acid derivative. Ibuprofen is chemically known as 2-(4-isobutylphenyl)-propionic acid. As used herein ibuprofen is understood to include 2-(4-isobutylphenyl)propionic acid as well as the pharmaceutically acceptable salts. Suitable ibuprofen salts include arginine, lysine, histidine, as well as other salts described in U.S. Pat. No. 4,279,926, 4,873,231, 5,424,075 and 5,510,385.

The amount of ibuprofen used in the present invention is a pharmaceutically effective amount. This amount can be from about 45 to about 75 % by weight, preferably about 50 to about 75 % by weight, most preferably about 70 % by weight, with respect to the total weight of the hard capsule.

What is meant by a "pharmaceutically active agent" is an agent (e.g., a compound) that is permitted or approved by the U.S. Food and Drug Administration, European Medicines Agency, or any successor entity thereof, for the oral treatment of a condition or disease. Suitable pharmaceutically active agents include, but are not limited to, analgesics, anti-inflammatory agents, antipyretics, antihistamines, antibiotics (e.g., antibacterial, antiviral, and antifungal agents), antidepressants, antidiabetic agents, antispasmodics, appetite suppressants, bronchodilators, cardiovascular treating agents (e.g., statins), central nervous system treating agents, cough suppressants, decongestants, diuretics, expectorants, gastrointestinal treating agents, anesthetics, mucolytics, muscle relaxants, osteoporosis treating agents, stimulants, nicotine, and sedatives.

Examples of suitable gastrointestinal treating agents include, but are not limited to: antacids such as aluminum-containing pharmaceutically active agents (e.g., aluminum carbonate, aluminum hydroxide, dihydroxyaluminum sodium carbonate, and aluminum phosphate), bicarbonate-containing pharmaceutically active agents, bismuth-containing pharmaceutically active agents (e.g., bismuth aluminate, bismuth carbonate, bismuth subcarbonate, bismuth subgallate, and bismuth subnitrate), calcium-containing pharmaceutically active agents (e.g., calcium carbonate), glycine, magnesium-containing pharmaceutically active agents (e.g., magaldrate, magnesium aluminosilicates, magnesium carbonate, magnesium glycinate, magnesium hydroxide, magnesium oxide, and magnesium trisilicate), phosphate-containing pharmaceutically active agents (e.g., aluminum phosphate and calcium phosphate), potassium-containing pharmaceutically active agents (e.g., potassium bicarbonate), sodium-containing pharmaceutically active agents (e.g., sodium bicarbonate), and silicates; laxatives such as stool softeners (e.g., docusate) and stimulant laxatives (e.g., bisacodyl); H2 receptor antagonists, such as famotidine, ranitidine, cimetadine, and nizatidine; proton pump inhibitors such as omeprazole, dextansoprazole, esomeprazole, pantoprazole, rabeprazole, and lansoprazole; gastrointestinal cytoprotectives, such as sucraflate and misoprostol; gastrointestinal prokinetics such as prucalopride; antibiotics for H. pylori, such as clarithromycin, amoxicillin, tetracycline, and metronidazole; antidiarrheals, such as bismuth subsalicylate, kaolin, diphenoxylate, and loperamide; glycopyrrolate; analgesics, such as mesalamine; antiemetics such as ondansetron, cyclizine, diphenyhydroamine, dimenhydrinate, meclizine, promethazine, and hydroxyzine; probiotic bacteria including but not limited to lactobacilli; lactase; racecadotril; and antiflatulents such as polydimethylsiloxanes (e.g., dimethicone and simethicone, including those disclosed in United States Patent Nos. 4,906,478, 5,275,822, and 6,103,260); isomers thereof; and pharmaceutically acceptable salts and prodrugs (e.g., esters) thereof.

Examples of additional suitable analgesics, anti-inflammatories, and antipyretics include, but are not limited to COX inhibitors such as celecoxib; acetaminophen; acetyl salicylic acid; acetic acid derivatives such as indomethacin, diclofenac, sulindac, and tolmetin; fenamic acid derivatives such as mefanamic acid, meclofenamic acid, and flufenamic acid; biphenylcarbodylic acid derivatives such as diflunisal and flufenisal; and oxicams such as piroxicam, sudoxicam, isoxicam, and meloxicam; isomers thereof; and pharmaceutically acceptable salts and prodrugs thereof.

Examples of antihistamines and decongestants, include, but are not limited to, bromopheniramine, chlorcyclizine, dexbrompheniramine, bromhexane, phenindamine, pheniramine, pyrilamine, thonzylamine, pripolidine, ephedrine, phenylephrine, pseudoephedrine, phenylpropanolamine, chlorpheniramine, dextromethorphan, diphenhydramine, doxylamine, astemizole, terfenadine, fexofenadine, naphazoline, oxymetazoline, montelukast, propylhexadrine, triprolidine, clemastine, acrivastine, promethazine, oxomemazine, mequitazine, buclizine, bromhexine, ketotifen, terfenadine, ebastine, oxatamide, xylomeazoline, loratadine, desloratadine, and cetirizine; isomers thereof; and pharmaceutically acceptable salts and esters thereof.

Examples of cough suppressants and expectorants include, but are not limited to, diphenhydramine, dextromethorphan, noscapine, clophedianol, menthol, benzonatate, ethylmorphone, codeine, acetylcysteine, carbocisteine, ambroxol, belladona alkaloids, sobrenol, guaiacol, and guaifenesin; isomers thereof; and pharmaceutically acceptable salts and prodrugs thereof.

Examples of muscle relaxants include, but are not limited to, cyclobenzaprine and chlorzoxazone metaxalone, orphenadrine, and methocarbamol; isomers thereof; and pharmaceutically acceptable salts and prodrugs thereof.

Examples of stimulants include, but are not limited to, caffeine.

Examples of sedatives include, but are not limited to sleep aids such as antihistamines (e.g., diphenhydramine), eszopiclone, and zolpidem, and pharmaceutically acceptable salts and prodrugs thereof.

Examples of appetite suppressants include, but are not limited to, phenylpropanolamine, phentermine, and diethylcathinone, and pharmaceutically acceptable salts and prodrugs thereof.

Examples of anesthetics (e.g., for the treatment of sore throat) include, but are not limited to dyclonine, benzocaine, and pectin and pharmaceutically acceptable salts and prodrugs thereof.

Examples of suitable statins include but are not limited to atorvastin, rosuvastatin, fluvastatin, lovastatin, simvustatin, atorvastatin, pravastatin and pharmaceutically acceptable salts and prodrugs thereof.

In one embodiment, the pharmaceutically active agent included within the tablet is selected from, aspirin, chlophedianol, dyclonine, and pharmaceutically acceptable salts and prodrugs thereof.

As discussed above, the pharmaceutically active agents of the present invention may also be present in the form of pharmaceutically acceptable salts, such as acidic/anionic or basic/cationic salts. Pharmaceutically acceptable acidic/anionic salts include, and are not limited to acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate, pantothenate, phosphate/diphospate, polygalacturonate, salicylate, stearate, subacetate, succinate, sulfate, tannate, tartrate, teoclate, tosylate and triethiodide. Pharmaceutically acceptable basic/cationic salts include, and are not limited to aluminum, benzathine, calcium, chloroprocaine, choline, diethanolamine, ethylenediamine, lithium, magnesium, meglumine, potassium, procaine, sodium and zinc.

As discussed above, the pharmaceutically active agents of the present invention may also be present in the form of prodrugs of the pharmaceutically active agents. In general, such prodrugs will be functional derivatives of the pharmaceutically active agent, which are readily convertible in vivo into the required pharmaceutically active agent. Conventional procedures for the selection and preparation of suitable prodrug derivatives are described, for example, in "Design of Prodrugs", ed. H. Bundgaard, Elsevier, 1985. In addition to salts, the invention provides the esters, amides, and other protected or derivatized forms of the described compounds.

Where the pharmaceutically active agents according to this invention have at least one chiral center, they may accordingly exist as enantiomers. Where the pharmaceutically active agents possess two or more chiral centers, they may additionally exist as diastereomers. It is to be understood that all such isomers and mixtures thereof are encompassed within the scope of the present invention. Furthermore, some of the crystalline forms for the pharmaceutically active agents may exist as polymorphs and as such are intended to be included in the present invention. In addition, some of the pharmaceutically active agents may form solvates with water (e.g., hydrates) or common organic solvents, and such solvates are also intended to be encompassed within the scope of this invention.

In one embodiment, the pharmaceutically active agent or agents are present in the capsule in a therapeutically effective amount, which is an amount that produces the desired therapeutic response upon oral administration and can be readily determined by one skilled in the art. In determining such amounts, the particular pharmaceutically active agent being administered, the bioavailability characteristics of the pharmaceutically active agent, the dose regime, the age and weight of the patient, and other factors must be considered, as known in the art.

### THE CAPSULE FILL

A variety of granular or particulate materials can be used to fill the capsules of the present invention. One form is a microbead or seamless capsules containing API's such as ibuprofen, naproxen or acetaminophen and combinations of these actives with other ingredients such as sleep aids, cough cold actives etc. are of interest since existing softgels are large and dissolve relatively slowly. The present invention incorporates more potent microbeads (which dissolve rapidly in water) in a perforated gelatin hardshell capsule. Examples of microbeads or seamless capsules useful in the present invention are disclosed in US Patent Nos. 9510609, 7842384 and 7255921.

Parameters of importance include concentration of API in the liquid fill of the microbeads. The amount of API is from about 40% to about 70%, preferably from about 50% to about 60% by weight of the liquid fill for Ibuprofen, Naproxen, and Acetaminophen (APAP). Other parameters of importance include optimal size of beads (dimension) to obtain maximum drug load in as small a capsule (size#) as possible; multi-colored beads for multiple API's in same capsule; percent of capsule area perforated relative to achieving best GI dissolution with least chance of dissolving in the oral cavity. In one embodiment one or more microbead may comprise a first active ingredient and a second microbead may comprise a second active ingredient.

In another embodiment, the capsules are filled with one or more sealed capsules. Examples of "sealed capsules" include those which are prefilled and sealed, comprise a sealing seam, and include an active ingredient.

In other embodiment, the capsules are filled with solid materials. Solid materials may include granules, solid beads, crystals, pellets, or mini-tablets. Granules may be prepared by such processes as high shear granulation, dry granulation, extrusion and spheronization, spray drying or spray congealing. Mini-tablets may be prepared by tablet compression. In some embodiments, one portion of the solid materials may comprise a first active ingredient and a second portion may comprise a second active ingredient.

In one embodiment, a portion of the solid material may be coated in order to modify the release mechanism of that portion. In one embodiment, the holes in the capsule facilitate a portion which disintegrates immediately and a second portion which is modified release.

As used herein, "modified release" shall apply to the altered release or dissolution of the active agent in a dissolution medium, such as gastrointestinal fluids. Types of modified release include, but are not limited to, sustained release or delayed release. In general, modified release tablets are formulated to make the active agents(s) available over an extended period of time after ingestion, which thereby allows for a reduction in dosing frequency compared to the dosing of the same active agent(s) in a conventional tablet. Modified release tablets also permit the use of active agent combinations wherein the duration of one pharmaceutically active agent may differ from the duration of another pharmaceutically active agent.

Examples of swellable, erodible hydrophilic materials for use as a release modifying excipient for use in the modified release coating include water swellable cellulose derivatives, polyalkylene glycols, thermoplastic polyalkylene oxides, acrylic polymers, hydrocolloids, clays, and gelling starches. Examples of water swellable cellulose derivatives include sodium carboxymethylcellulose, cross-linked hydroxypropylcellulose, hydroxypropyl cellulose (HPC), hydroxypropylmethylcellulose (HPMC), hydroxyisopropylcellulose, hydroxybutylcellulose, hydroxyphenylcellulose, hydroxyethylcellulose (HEC), hydroxypentylcellulose, hydroxypropylethylcellulose, hydroxypropylbutylcellulose, and hydroxypropylethylcellulose. Examples of polyalkylene glycols include polyethylene glycol. Examples of suitable thermoplastic polyalkylene oxides include poly (ethylene oxide). Examples of acrylic polymers include potassium methacrylatedivinylbenzene copolymer, polymethylmethacrylate, and high-molecular weight cross-linked acrylic acid homopolymers and copolymers.

Suitable pH-dependent polymers for use as release-modifying excipients for use in the modified release coating include: enteric cellulose derivatives such as hydroxypropyl methylcellulose phthalate, hydroxypropyl methylcellulose acetate succinate, and cellulose acetate phthalate; natural resins such as shellac and zein; enteric acetate derivatives such as polyvinylacetate phthalate, cellulose acetate phthalate, and acetaldehyde dimethylcellulose acetate; and enteric acrylate derivatives such as for example polymethacrylate-based polymers such as poly(methacrylic acid, methyl methacrylate) 1:2 (available under the tradename EUDRAGIT S) and poly(methacrylic acid, methyl methacrylate) 1:1 (under the tradename EUDRAGIT L).

In one embodiment, the microbead or solid fill is coated with a reverse enteric polymer in order to prevent disintegration in the mouth through the capsule openings, and disintegrate at stomach pH.

In another embodiment, an additional material or materials are added to the capsule fill to further facilitate disintegration. Such materials may comprise disintegrants such as but not limited to sodium starch glycolate, crospovidone, croscarmellose, derived starches and calcium silicate, added at a level of about 0.05 to about 10 percent by weight of the dosage form. In one embodiment, the additional material is granulated or compressed into slugs or tablets at a larger size in order to prevent falling from the capsule holes. In another embodiment, the additional material is comprised of an acid base couple, which facilitates the release of gas or carbon dioxide upon contact with water. Suitable acid-base couples are typically comprised of combination of an organic acid such as citric acid, malic acid, ascorbic acid or fumaric acid and a base such as sodium bicarbonate. The total level of the acid-base couple is about 0.5 to about 20 percent by weight of the dosage form.

### THE CAPSULE PERFORATIONS

The capsule is perforated to facilitate the rapid dissolution once in the GI tract but not dissolve in the oral cavity. The capsule protects the particulate fill but does not impede their rapid dissolution in the stomach. The dosage form will rupture in significantly shorter time than the traditional hard or soft capsule systems. In certain preferred embodiments of the invention the dosage form further comprises one, or more preferably a plurality of perforations in the capsule. The perforations may be of any shape and size, and may optionally be arranged in a pattern. In embodiments in which the perforations are made by laser ablation, the width or diameter of the smallest opening is typically at least 1 - 2 times the wavelength of light provided by the laser employed. At least a portion of the perforation may be large enough to be seen with the unaided human eye, ranging in width or diameter from about 400 nanometers to as much as any dimension that can maintain the integrity of the capsule. The perforations will have minimum dimension of least about 0.5 mm, e.g. at least about 0.75 mm, or at least about 1.0 mm. Typically, visible perforations will have a maximum width or diameter of not more than the width of the capsule, for example not more than about 6.5 millimeters, or not more than about 3.5 millimeters, or not more than about 2.5 millimeters. Alternatively, some or all the perforations may be microscopic in size, ranging from about 1 to less than about 400 nanometers in width or diameter. In embodiments in which some or all the perforations are invisible to the unaided human eye, a plurality of perforations may be arranged in a pattern that creates a weak spot in the capsule, which facilitate disintegration. In one embodiment, wherein the perforation is round, the size of the perforation or hole is from about 0.1mm to about 5.0mm, or about 0.5mm to about 3.0mm. In an embodiment, wherein the perforation is oblong, the diameter of the width of the perforation is from about 0.1mm to about 5.0mm., or about 0.5mm to about 3.0mm.

As shown in Fig. 1, capsules can have a series of evenly spaced rows of holes. However, the pattern of the holes and the number can vary. For example, four rows of three or four holes evenly spaced along capsule body diameter and a single hole at the crown of capsule body can be used. Or any number of holes, shapes and sizes can be used as long at the integrity of the capsules is maintained so it enters the gastrointestinal system intact. In one embodiment, the openings or holes are elongated, or slits which extend across the face of the capsule, or in the opposite direction of the elongation of the capsule. In one embodiment, the width of the elongated hole is less than the diameter of the filled microcapsule or solid fill material. In another embodiment, the capsule holes are formed in the shape of indicia such as numbers or letters, which may indicate product type, brand, trade dress or dosage.

Other ways to make the perforations include mechanical means such as dipping pins in the gelatin during the manufacture of the capsule. Drilling holes in the capsule after it is formed is another way to make the perforations.

The capsule shells of the present invention may be comprised of different materials. Materials include gelling components such as gelatin, gellan gum, and carrageenan. Materials within the capsule shell may also include starches, modified starches, polymers such as hypromellose and hydropxypropylcellulose, and plasticizers such as glycerin, propylene glycol, polyethylene glycol, castor oil, and triethylcitrate.

In one embodiment, more than one type of capsule fill may be added to the gelatin capsule. In one embodiment one type of capsule fill is of one size, while a second type of capsule fill is of another size to fill the interstitial spaces between the larger capsule fill.

In a variation of the embodiment above, capsule fill may contain a second active ingredient.

The perforations in the capsule may be of any shape which does not allow for the capsule fill to fall out (or separate) from the form. In one embodiment, the diameter of the openings is at least 10% smaller, e.g. at least 5% smaller than the diameter of the capsule fill. The openings may be circular, in which they are smaller than the capsule fill. These may also be elongated openings which extend across the small axis of the capsule form, or at an angle at least up to 90 degrees perpendicular to the small axis. In one embodiment, there are openings on both halves of a capsule shell. In another embodiment, the openings are present on only one-half of the capsule shell

Various methods can be used to seal the hard gelatin capsules according to the invention.

The banding of hard gelatin capsules is well-known in the art. The capsules are first rectified and then passed once or twice over a wheel that revolves in a gelatin bath. An amount of gelatin is picked up by the serrated wheel and applied to the junction of the cap and body. The capsules remain in individual carries for drying. The sealing band may be made up of gelatin or other water-soluble film forming polymers such as but not limited to hypromellose; hydroxypropylcellulose; polyvinyl pyrrolidone, gellan gum, microcrystalline cellulose, carrageenan; polyvinyl alcohol, polyethylene glycol and related co-polymers.

### EXAMPLES

### Example 1: Preparation of Microbeads containing Menthol

### Part A: Preparation of Bead Coating Layer

1. The outer coating layer is prepared using the ingredients in Table 1. The purified water is heater to about 30oC.
2. The glycerin, isomalt are added and mixed.
3. The gelatin is then added, and the solution is raised to 60°C and mixed for at least one hour until dissolved

### Part B: Preparation of Fill Solution and creation of capsules

1. The vegetable oil is melted at about 40-55°C.
2. Menthol is then added until dissolved.
3. Silicon dioxide is then added while mixing and homogenized for about 20 minutes while mixing at about 30-45°C.
4. The materials are extruded through a double nozzle and dropped into a cooling solution containing vegetable oil.
5. The capsules are collected and the residual oil is drained.

**Table 1**

| **Material** | **mg/Capsule** | **% W/W** | **Batch wt (g)** |
|---|---|---|---|
| Coating Materials | | | |
| Gelatin^{a} | 3.6 | 60.0 | 60.0 |
| Glycerin | 1.2 | 20.0 | 20.0 |
| Isomalt | 1.2 | 20.0 | 20.0 |
| Purified Water | ----- | ----- | 400.0 |
| TOTAL | 6.00 | 100.0 | 500.0 |
| | | | |
| | | | |
| Core Materials | | | |
| Vegetable Oil | 92.00 | | |
| Menthol | 7.50 | | |
| Silicon Dioxide | 0.50 | | |
| | | | |
| **TOTAL** | 200.0 | 100.0 | 50.0 |

### Example 2: Preparation of Filled Capsule Containing Microbeads

Three of the prepared microbeads from Example 1 are added to a perforated gelatin capsule. The beads were put in capsule size zero.

The perforations are made by a DPSS Laser - SAML-03-N0500, 3 Watt, manual operation with variable time as capsules are rotated while on a mandrel to orient to laser.

Holes were drilled using hole diameters of 0.5mm and 1.0 mm with 4 rows of 3 or 4 holes evenly spaced across the capsule body diameter and a single hole at the crown of capsule body.

### Example 3: Fill Material using Compressed Granules (Slugs)

Compressed granules were prepared for inclusion in the perforated capsule. The materials are described in Table 2.

**Table 2: Materials for Compressed Granules**

| | **Ingredients** | **Batch weight (g)** | **Percentage** |
|---|---|---|---|
| 1 | Acetaminophen (Compap^{®} 90%)^{a} | 270.0 | 90.0 |
| 2 | Crospovidone (Plasdone S 630)^{b} | 9.0 | 3.0 |
| 3 | Microcrystalline Cellulose (Avicel PH 101)^{c} | 18.0 | 6.0 |
| 4 | Magnesium stearate | 3.0 | 1.0 |
| | **Total** | **300.0** | **100.0** |

| | | | |
|---|---|---|---|
| a: Commercially available as Compap from the Mallicrodt Corporation b: Commercially available as Plasdone S 630 from the Ashland Corporation c: Commercially available as Avicel PH101 from the FMC Corporation | | | |

### Blending and Compression Procedure:

1. All the ingredients were screened through #20 mesh stainless steel screen
2. All the ingredients were blended for 5 minutes in a turbula mixer
3. 1.5 g tablet slugs were manufactured at a 22kN (5000 lbs) compression force using 11/16 flat faced beveled (FFBE) tooling
4. The tablet slugs are then triturated into large granules using mortar and pestle
5. Resultant large granules were screened through #8 mesh stainless steel screen to remove any fine particles
6. The large granules are then filled into perforated capsules from Example 1.

## Claims

1. A perforated hard capsule comprising at least one perforation having a first dimension and containing at least one particle with a dimension larger than the perforation in the capsule wherein the capsule and the at least one particle are not dissolvable in the mouth.

2. The perforated hard capsule according to claim 1 comprising a plurality of perforations.

3. The perforated hard capsule according to claim 1 wherein the first dimension of the at least one perforation is from 0.1 mm to 5.0 mm.

4. The perforated hard capsule according to claim 2 wherein the first dimension of the plurality of perforations is from 0.5 mm to 3.0 mm.

5. The perforated hard capsule according to any preceding claim wherein the at least one particle is a microbead, a seamless capsule, a sealed capsule or a coated solid material and contains a pharmaceutically active ingredient.

6. The perforated hard capsule according to claim 1 wherein the particles are liquid filled microbeads.

7. The perforated hard capsule according to claim 6 wherein the microbeads are made from gelatin.

8. The perforated hard capsule according to claim 6 wherein the liquid comprises a pharmaceutically active agent.

9. The perforated hard capsule according to claim 8 wherein the pharmaceutically active agent is ibuprofen.

10. The perforated hard capsule according to claim 8 wherein the pharmaceutically active agent is acetaminophen.

11. The perforated hard capsule according to claim 1 wherein the particles are compressed granules.

12. The perforated hard capsule according to claim 11 wherein the compressed granule comprises a pharmaceutically active agent.

13. The perforated hard capsule according to claim 12 wherein the pharmaceutically active agent is ibuprofen.

14. The perforated hard capsule according to claim 12 wherein the pharmaceutically active agent is acetaminophen.

15. The perforated hard capsule according to any previous claim, wherein the at least one particle is a microbead or solid material which is coated with a reverse enteric polymer.

## Patentansprüche

1. Perforierte Hartkapsel umfassend wenigstens eine Perforation mit einer ersten Abmessung und enthaltend wenigstens ein Partikel mit einer Abmessung, die größer als die Perforation in der Kapsel ist, wobei die Kapsel und das wenigstens eine Partikel nicht im Mund löslich sind.

2. Perforierte Hartkapsel nach Anspruch 1, umfassend eine Vielzahl von Perforationen.

3. Perforierte Hartkapsel nach Anspruch 1, wobei die erste Abmessung der wenigstens einen Perforation von 0,1 mm bis 5,0 mm beträgt.

4. Perforierte Hartkapsel nach Anspruch 2, wobei die erste Abmessung der Vielzahl von Perforationen von 0,5 mm bis 3,0 mm beträgt.

5. Perforierte Hartkapsel nach einem der vorstehenden Ansprüche, wobei das wenigstens eine Partikel ein Mikrokügelchen, eine nahtlose Kapsel, eine versiegelte Kapsel oder ein beschichtetes festes Material ist und einen pharmazeutischen Wirkstoff enthält.

6. Perforierte Hartkapsel nach Anspruch 1, wobei die Partikel flüssigkeitsgefüllte Mikrokügelchen sind.

7. Perforierte Hartkapsel nach Anspruch 6, wobei die Mikrokügelchen aus Gelatine bestehen.

8. Perforierte Hartkapsel nach Anspruch 6, wobei die Flüssigkeit einen pharmazeutischen Wirkstoff umfasst.

9. Perforierte Hartkapsel nach Anspruch 8, wobei der pharmazeutische Wirkstoff Ibuprofen ist.

10. Perforierte Hartkapsel nach Anspruch 8, wobei der pharmazeutisch aktive Wirkstoff Acetaminophen ist.

11. Perforierte Hartkapsel nach Anspruch 1, wobei die Partikel komprimierte Granulatkörner sind.

12. Perforierte Hartkapsel nach Anspruch 11, wobei das komprimierte Granulat einen pharmazeutischen Wirkstoff umfasst.

13. Perforierte Hartkapsel nach Anspruch 12, wobei der pharmazeutische Wirkstoff Ibuprofen ist.

14. Perforierte Hartkapsel nach Anspruch 12, wobei der pharmazeutisch aktive Wirkstoff Acetaminophen ist.

15. Perforierte Hartkapsel nach einem vorstehenden Anspruch, wobei das wenigstens eine Partikel ein Mikrokügelchen oder festes Material ist, das mit einem umgekehrt enterischen Polymer beschichtet ist.

## Revendications

1. Capsule dure perforée comprenant au moins une perforation ayant une première dimension et contenant au moins une particule ayant une dimension plus grande que la perforation dans la capsule, la capsule et l'au moins une particule n'étant pas dissoutes dans la bouche.

2. Capsule dure perforée selon la revendication 1, comprenant une pluralité de perforations.

3. Capsule dure perforée selon la revendication 1, la première dimension de l'au moins une perforation étant de 0,1 mm à 5,0 mm.

4. Capsule dure perforée selon la revendication 2, la première dimension de la pluralité de perforations étant de 0,5 mm à 3,0 mm.

5. Capsule dure perforée selon l'une quelconque des revendications précédentes, l'au moins une particule étant une microbille, une capsule sans soudure, une capsule scellée ou une matière solide enrobée et contenant un ingrédient pharmaceutiquement actif.

6. Capsule dure perforée selon la revendication 1, les particules étant des microbilles remplies de liquide.

7. Capsule dure perforée selon la revendication 6, les microbilles étant fabriquées à partir de gélatine.

8. Capsule dure perforée selon la revendication 6, le liquide comprenant un agent pharmaceutiquement actif.

9. Capsule dure perforée selon la revendication 8, l'agent pharmaceutiquement actif étant l'ibuprofène.

10. Capsule dure perforée selon la revendication 8, l'agent pharmaceutiquement actif étant l'acétaminophène.

11. Capsule dure perforée selon la revendication 1, les particules étant des granules comprimés.

12. Capsule dure perforée selon la revendication 11, le granule comprimé comprenant un agent pharmaceutiquement actif.

13. Capsule dure perforée selon la revendication 12, l'agent pharmaceutiquement actif étant l'ibuprofène.

14. Capsule dure perforée selon la revendication 12, l'agent pharmaceutiquement actif étant l'acétaminophène.

15. Capsule dure perforée selon l'une quelconque des revendications précédentes, l'au moins une particule étant une microbille ou un matériau solide qui est enrobé d'un polymère entérique inverse.
